# EUROPEAN PATENT SPECIFICATION

(11) **EP 4 528 272 B1**
(45) Date of publication and mention of the grant of the patent: **22.04.2026**
(21) Application number: 23198984.9
(22) Date of filing: 22.09.2023
(51) Int. Cl.: G01N 13/00, G01N 33/15, G01N 35/10

(54) **DISSOLUTION TESTING APPARATUS AND METHOD FOR OPEN OR CLOSED LOOP CONFIGURATION**
AUFLÖSUNGSPRÜFGERÄT UND VERFAHREN FÜR OFFENE ODER GESCHLOSSENE REGELKREISKONFIGURATION
APPAREIL D'ESSAI ET PROCÉDÉ DE DISSOLUTION POUR CONFIGURATION EN BOUCLE OUVERTE OU FERMÉE

(43) Date of publication of application: 26.03.2025
(73) Proprietor: SOTAX AG, 4147 Aesch (CH)
(72) Inventor: Benz, Rolf, Aesch 4147 (CH); Fraga, Rui, 4147 Aesch (CH); Magnier, Michel, 4147 Aesch (CH); Kazmierczak, Damian, 4147 Aesch (CH); Hänggi, Steven, 4147 Aesch (CH); Kalbermatten, Gilles, 4147 Aesch (CH)
(74) Representative: Braunpat AG

(56) References cited:
- WO-A1-83/03901
- CN-A- 105 784 953
- US-A- 4 678 639
- US-A- 4 708 023
- US-A- 6 060 024
- US-A1- 2003 086 823
- US-A1- 2020 150 099
- US-A1- 2021 011 038

## Description

### Technical Field

The invention relates to a dissolution testing apparatus and method for drug release testing and dissolution testing, especially to a flow-through cell instrument or apparatus having a module for open or closed mode operation. The invention particularly relates to dissolving and testing a dissoluble substance.

### Background of the invention

Dissolution testing is a compendial in vitro test for example used in pharmaceutical research and development to characterize the release rate of an Active Pharmaceutical Ingredient (API) from a formulated dosage form, under reproducible conditions, in correlation with its in vivo performance. Once an innovator product dossier has been approved by the addressed market authorities, dissolution testing is then used to assess batch-to-batch consistency of the manufactured product according to its submitted specification and according to general testing conditions described by authorities. Dissolution is also used by generic companies to establish the bioequivalence of their products, without redoing clinical studies initially done by the innovator: doing so they obtain a biowaiver.

Pharmaceutical formulations include granules, tablets, capsules, gels, creams, ointments, suspensions, suppositories, patches, microspheres, implants and other long acting injectables as micro and nano suspensions and others. "Dissolution testing" is a term used for solid dosage forms and the more general term: "Drug release testing" is used for other dosage forms, including coated medical devices. Outside of the pharmaceutical segment, dissolution testing apparatuses are also used in applications such as nicotine release from smokeless tobacco products and for persistence tests of stone wool fibers in lung fluids. The first interest of a dissolution testing apparatus in form of a flow-through cell dissolution instrument lies in its flexibility as the cell and its inside arrangement can be adapted to the tested formulation structure and challenges.

Dissolution testing modalities for solid dosage forms and apparatus involved are described in a general dissolution chapter harmonized between European, US and Japanese Pharmacopeias: the USP <711>. A flow-through cell dissolution is also described as method 6 in the Chinese Pharmacopeia.

Standardized methods use for example a paddle apparatus for stirring substances in a container as for example described in EP 0746759 A1, a basket apparatus for holding the substance in a basket as for example described in US 5816701 A, or a flow-through apparatus providing dissolution medium flowing through a cell containing the substance as for example described in WO 2013/003518 A1. The dimensions and tolerances of such apparatuses are specified precisely. Critical test parameters that have to be monitored periodically during use include for example volume and temperature of the dissolution medium, rotation speed and flow rate of medium.

The flow-through apparatus is used for a wide range of objects to be tested. The flow-through cell dissolution principle consists in flowing dissolution media at defined temperature and flow rate onto a cell containing the dosage form to be tested. The total volume of dissolution medium is based on the solubility of the active pharmaceutical ingredient, respectively substance, to be dissolved and the test duration is ideally correlated to the In vivo performance of the tested product.

Dissolution testing apparatuses are generally composed of multiple channels to allow the simultaneous testing of for example seven channels (six samples and a reference). Therefore, the seven channels have to be qualified in a documented procedure (Installation Qualification, Operational Qualification) prior to any use of the apparatus to prove that they allow an equivalent testing on all channels, as finally samples will be all compared to specifications because they are representing the entire manufactured batch. A flow-through cell dissolution testing apparatus comprises reservoir(s) of medium, a multi channels pump system, a dissolution module (containing the cells) and module allowing fraction collection or sampling or directly analysing the samples on-line.

A flow-through cell dissolution apparatus can be utilised in two different configurations/modes.

In the open mode, the channels are receiving dissolution medium simultaneously from a unique reservoir. The medium is flowed by individual pump channels into the flow-through cells, filtered, and the individual eluates, respectively solutions, are then collected or analysed. The dosage form is immersed in media which is always renewed in the cell at a speed defined by the pump flow rate setting. In current systems the sample fraction collected have to be homogenised and a smaller sample in volume has to be withdrawn from the fraction, to be analysed. During the test, if different reservoirs have been connected to a medium selector, it will be possible to change the source of medium (for all cells) at a defined time. As the medium used can have different pH this medium change can be used to mimic the pH change happening in the Gastro-Intestinal tract. Therefore, the dosage forms in the cells can be subject to an equivalent pH change than the one operated in vivo.

In the closed mode, medium volumes are stored in individual reservoirs for each channel. Each medium reservoir is connected to an individual pump channel which is connected to an individual flow-through cell, and finally connected back to the medium reservoir. This loop makes the media flow onto the cell, being filtered and coming back to the reservoir in a stirred manner. The dissolved active ingredient which is released from the dosage form and solubilized passes through a filter and accumulates in the stirred reservoir from where it can be sampled. In current systems, the test is only done at a unique pH.

A dissolution testing apparatus with a closed loop configuration is for example described in WO 2021/076558 A1. The apparatus includes a fluid reservoir for storing a medium comprising a solvent for extracting substances of a pharmaceutical dosage form as well as for receiving a substance solution of the medium and the dissolved substance. The dosage form is placed in a flow-through cell which is connected to the fluid reservoir and a pump delivers fluid from the reservoir to the flow-through cell. A multiport manifold is used to circulate fluid between the reservoir and the cell. A magnetic stirring mechanism is used to stir the substance solution in the fluid reservoir.

US4,678,639 discloses an apparatus for periodically monitoring the composition of a plurality of samples and includes a plurality of stationary flow-through sample vials through which a sample fluid is periodically passed. A sampling probe withdraws fluid from the vial for subsequent analysis.

However, most apparatuses are designed as an open system or a closed system, and they are not set up to easily switch between open and closed modes. The current apparatuses require unplugging and re-plugging of tubing and sampling modules exchange to switch from an open to a closed configuration.

It is an object of the invention to provide a dissolution testing apparatus and dissolution testing method in accordance with independent claims 1 and 12 respectively which allow quick and simple switching between open and closed loop configurations, which enhance flexible adjustment of the apparatus to improve the reliability and reproducibility of testing conditions and methods, and enable fast and accurate testing of substance solution samples.

### Summary of the invention

These and other objects, which will appear from the description below, are achieved by a dissolution testing apparatus and dissolution testing method as set forth in the appended independent claims. Preferred embodiments are defined in the dependent claims.

The dissolution testing apparatus for testing a dissoluble substance dissolved in a dissolution medium according to the present invention comprises at least one dissolution medium reservoir, at least one flow-through cell for receiving a dissoluble substance to be tested, which is connected to the dissolution medium reservoir, and at least one pump device for pumping dissolution medium through the at least one flow-through cell. The at least one flow-through cell comprises a cell inlet for the dissolution medium and a cell outlet for a substance solution containing dissolution medium and dissolved substance. Further, the at least one flow-through cell is designed to accommodate a specific form of the dissoluble substance, like a capsule or tablet etc. The flow-through cell may comprise a holding structure for positioning the dissoluble substance within the cell.

According to the present invention the dissolution testing apparatus comprises two sampling vessels for each flow-through cell, that are connectable and dis-connectable to the flow-through cell. The sampling vessels sample a substance solution containing dissolution medium and dissolved substance. Further, the dissolution testing apparatus comprises an inlet fluid manifold for selectively connecting the flow-through cell to one of the at least two sampling vessels, an outlet fluid manifold for selectively connecting one of the at least two sampling vessel to a discharge line, at least one testing device for testing the substance solution of the selected sampling vessel, and a switch valve system for selectively connecting the discharge line to a cell inlet line of the flow-through cell or an extraction line. The dissolution testing apparatus may have several flow-through cells that are for example arranged in parallel to each other. Optionally, the dissolution testing apparatus could have more than two sampling vessels for each flow-through cell.

According to the present invention the sampling vessels for sampling a substance solution emitted from the flow-through cell may serve for collecting substance solution as well as for temporarily storing it. The at least two sampling vessels are an integral component of the fluid flow through the apparatus and increase the operational flexibility of the dissolution testing apparatus. When exiting the sampling vessels, the substance solution may be re-circulated to the flow-through cell, extracted to a sample solution vessel for external handling or even to waste.

The inlet fluid manifold is designed to connect just one of the at least two sampling vessels to the flow-through cell and to switch between the sampling vessels. Thus, there is just one sampling vessel selected to be connected to the flow-through cell at a given point in time. Likewise, the outlet fluid manifold is designed to connect just one of the at least two sampling vessels to the discharge line and to switch between the sampling vessels. Thus, there is just one sampling vessel selected to be connected to the flow-through cell and filled with substance solution, while the same or another one of the at least two sampling vessels can be connected to the discharge line at a given point in time.

In the context of the present specification of the invention, the expression "connected to" shall be understood in that two components like the cell, the sampling vessel, the discharge line, the inlet line, etc. are in fluid communication such that a fluid like the dissolution medium, the substance solution, a cleaning medium, etc. can flow from one component to the other and through the components, respectively. Accordingly, the expressions "connectable" and "dis-connectable" shall be understood in that the fluid communication can be established, respectively interrupted, for example by a valve, a fluid splitter, liquid distributors and the like.

According to the dissolution testing method for testing a dissoluble substance dissolved in a dissolution medium of the present invention, the inlet fluid manifold, the outlet fluid manifold and/or the switch valve system establish selective fluid connections such that the dissolution testing apparatus is operated in an open loop mode by connecting the discharge line to the extraction line or alternatively the apparatus is operated in a closed loop mode by connecting the discharge line to the cell inlet line. The switch valve system is coordinated with the inlet fluid manifold and the outlet fluid manifold to direct the substance solution exiting the selected sampling vessel according to a fluid circulation back to the flow-through cell for closed loop testing or a fluid flow extracting the substance solution for open loop testing.

The dissolution testing apparatus according to the invention broadens the options of flexibly adjusting the apparatus to in vivo conditions by customizing a flow of dissolution medium passing the dissoluble substance and ensuring consistent flow conditions when needed. The dissolution testing apparatus provides consistent dissolution quality for dissolving diverse forms of substances, particularly of drug substances. The dissolution testing apparatus enables versatile and automated dissolution testing and supports precise and predictive test results. The dissolution testing apparatus according to the invention creates a module - based on two sampling vessels for each flow-through cell - which allows a simple switch from open to closed configurations, a straight transfer from fraction collection to sampling process and a pH change available also in closed systems.

In one embodiment of the dissolution testing apparatus at least the inlet fluid manifold, the outlet fluid manifold and the switch valve system are connected to a controller for controlling selective fluid connection between the flow-through cell, the sampling vessels, the discharge line, the cell inlet line, and the extraction line. For example, the controller is realized by an electronic control system which is designed to electronically command operation of the inlet fluid manifold, the outlet fluid manifold, and the switch valve system, which in turn are configured with electronic steering components communicating with the electronic control system. Further, the controller may comprise a processing unit for processing testing protocols defining testing procedures, input means for entering testing information data, display means for displaying testing information, testing results, etc., and a storage unit for storing testing protocols, information data, test results, etc.

In one embodiment of the dissolution testing apparatus according to the invention at least one testing device is arranged at the discharge line and/or at the at least one sampling vessel for testing substance solution in the discharge line and/or in the sampling vessel. Thus, the substance solution can be tested shortly after the substance has dissolved in the dissolution medium and even while the substance solution is still processed in the dissolution testing apparatus. For example, the testing device can be an optical testing device, like a UV spectrophotometer. Alternatively, the substance solution can be extracted from the fluid flow and stored in a sample solution vessel for further sample analysis, as commonly known.

In an example embodiment of the dissolution testing apparatus according to the present invention the inlet fluid manifold comprises an inlet port in fluid communication with a substance solution conduit of the flow-through cell and an outlet port for each of the sampling vessels, wherein the outlet port is in fluid communication with a sampling vessel inlet line. The outlet fluid manifold comprises an inlet port for each of the sampling vessels, which are in fluid communication with a sampling vessel outlet line of the respective sampling vessel, and an outlet port in fluid communication with the discharge line. The substance solution enters the inlet fluid manifold through the inlet port and is directed to the outlet port associated to the selected sampling vessel to connect the flow-through cell to the selected sampling vessel. Once a first selected sampling vessel is filled, for example according to testing requirements, the inlet fluid manifold switches to another outlet port to select another, i.e. a second sampling vessel. The outlet fluid manifold opens a manifold inlet port of one of the sampling vessels for receiving substance solution, for example from the first sampling vessel while the second sampling vessel is filled, and directs it to the outlet port for the discharge line or another outlet port. Once the substance solution of that first sampling vessel is processed, the outlet fluid manifold can address the second sampling vessel.

The switch valve system is for example a valve unit, wherein the discharge line, the extraction line and the cell inlet line are connected to this valve unit. Also, the switch valve system may include several switch valves and at least one re-circle line or a bypass line to re-direct the substance solution from the discharge line to the cell inlet line. The inlet fluid manifold, the outlet fluid manifold and the switch valve system may be used to control the flow rate into and through the sampling vessels, for example according to standard testing protocols.

In a still further embodiment of the dissolution testing apparatus according to the present invention a stirring loop is provided at a sampling vessel outlet line, comprising a pump for pumping substance solution through the stirring loop and back into the sampling vessel. For example, the stirring loop may be a fluid line comprising the pump and merging back into the sampling vessel. The stirring loop allows for flexible and uniform mixing of the substance solution of a sampling vessel. Advantageously, the stirring loop may be connected to the outlet fluid manifold. The outlet fluid manifold may comprise a stirring port for connecting the at least one sampling vessel to the stirring loop.

In another embodiment of the dissolution testing apparatus the outlet fluid manifold may comprise a waste port for connecting the at least two sampling vessels to a waste line. The waste line is for example useful for discarding excess substance solution, when the apparatus operates in an open loop mode. Further, a cleaning system may be provided for cleaning the at least two sampling vessels. The cleaning system may comprise a cleaning medium reservoir outside the at least two sampling vessels and a vessel inlet, like a spray nozzle, at each of the sampling vessels. The cleaning medium can be water for example or any other suitable cleaning fluid. The cleaning system is connected to the inlet fluid manifold to connect the cleaning system to the at least two sampling vessels, respectively their spray nozzles. The cleaning medium can be circulated by the stirring loop and discarded from the sampling vessel through the waste line.

Advantageously, the dissolution medium reservoir can be connected and disconnected to the flow-through cell by the switch valve system. The switch valve system may connect the dissolution medium reservoir to the in-flow conduit leading to the cell inlet. Thus, the dissolution medium may be supplied to the flow-through cell as needed for dissolution. In one example, the dissolution medium reservoir comprises a media selector providing several different solvents and a media manifold, which is connectable to the flow-through cell by the switch valve system or even is an integral part of the switch valve system. Thus, different solvents or additives can be added to the dissolution medium before entering the flow-through cell and the dissolution medium conditions can be flexibly adjusted to specific testing conditions. Particularly, a pH value of the dissolution medium in the cell can be adjusted as required.

In a further example embodiment, the dissolution testing apparatus may comprise a heating module for heating dissolution medium at an in-flow conduit leading to the flow-through cell. Thus, the dissolution medium can be heated according to requirements of a testing protocol or according to in vitro conditions as desired. Further, the at least one flow-through cell and/or the at least two sampling vessels can be arranged in a heating channel for heating the substance solution in the cells or the vessels, respectively. Thus, the dissolution medium and/or the substance solution can be heated according to requirements of a testing protocol or according to in vitro conditions as desired.

As mentioned above, the dissolution testing apparatus can advantageously be operated in an open loop mode or in a closed loop mode by switching connection of the at least two sampling vessels between extraction line and cell inlet line. In one variant of the dissolution testing method according to the present invention, the at least two sampling vessels are alternately connected to the flow-through cell and the discharge line such that a continuous substance solution flow is exiting the flow-through cell. This continuous substance solution flow runs through the substance solution conduit connecting the flow-through cell and the inlet fluid manifold. The fluid manifolds connected to the sampling vessels control the inflow and outflow of the substance solution such that a first sampling vessel is filled while a fraction of substance solution in a second sampling vessel is processed for analysis and emptied for receiving a subsequent fraction of substance solution. The second vessel buffers the substance solution while the continuous substance solution flow is directed to the first sampling vessel. Vice versa, the fraction in the first sampling vessel is processed for analysis while the second vessel is filled. Thus, the at least two sampling vessels take turns in receiving substance solution from the flow-through cell and also take turns in being discharged. This process is particularly useful for an open loop mode and allows for fast substance solution processing. If necessary, more than two sampling vessels can be used for processing and preparing the substance solution for analysis or extraction of the apparatus.

In an advantageous variant of the dissolution testing method fractions of the substance solution are tested in the sampling vessels. For example, optical detectors can analyse the substance solution within the sampling vessel while the solution stays in the vessel or is stirred in the stirring loop. This process speeds up the testing of the substance solution. Alternatively, the fractions of substance solution can be tested by a testing device arranged in the discharge line, while the substance solution flows therethrough. Or the fraction can be tested after being extracted from the discharge line.

In an example variant of the dissolution testing method that operates in a close loop mode the flow-through cell is connected to one of the at least two sampling vessels and the discharge line is connected to the cell inlet line, and several samples are sampled consecutively and tested by the testing device. In closed loop mode the sampling vessel can be used as dissolution medium reservoir, wherein the dissolution volume can be larger than the cell volume. Advantageously, the substance solution is tested before it enters the cell inlet line. For example, a testing device is arranged in the discharge line or a re-circle line of the switch valve system. Thus, for the closed loop mode only one of the sampling vessels is used. The invention is here to allow a switch between the two vessels to allow a media change (and therefore a pH change) in a closed system.

The dissolution testing apparatus and the dissolution testing method according to the present invention enable versatile and automated flow-through cell dissolution testing and support precise and reproducible test results. The operability between the different configurations is enhanced and secured and the transfer from fractions to samples is automated and therefore traceable.

### Brief description of the drawings

Preferred embodiments of the invention will be described in the accompanying drawings, which may explain the principles of the invention but shall not limit the scope of the invention. The drawings illustrate:
- Fig. 1: an overview of operation modules of a dissolution testing apparatus according to the present invention,
- Fig. 2: a schematical illustration of substance solution flow in a sampling module of the dissolution testing apparatus,
- Fig. 3: a schematical diagram of an open/closed loop configuration of a dissolution testing apparatus according to the present invention,
- Fig. 4: a schematical diagram of a closed loop configuration of a dissolution testing apparatus according to the present invention, and
- Fig. 5a & 5b: an assembled view and an exploded view of a flow-through cell as used in a dissolution testing apparatus of the present invention.

### Detailed description of a preferred embodiment

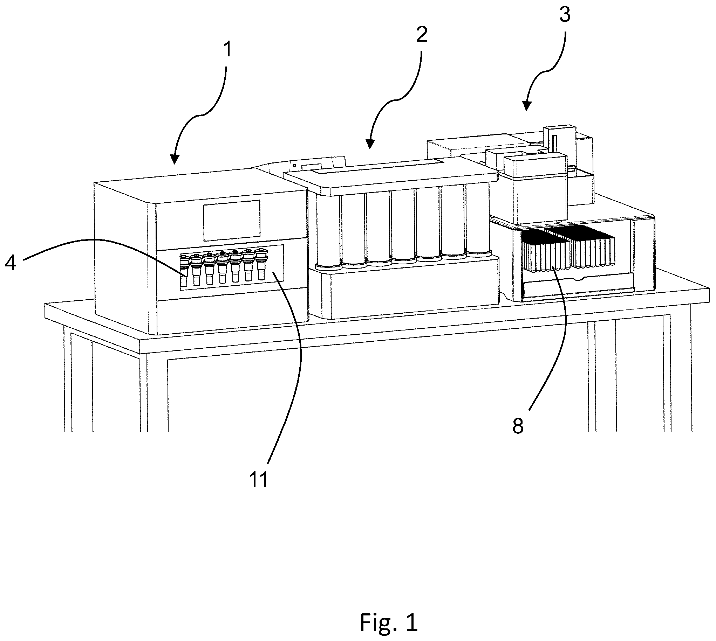

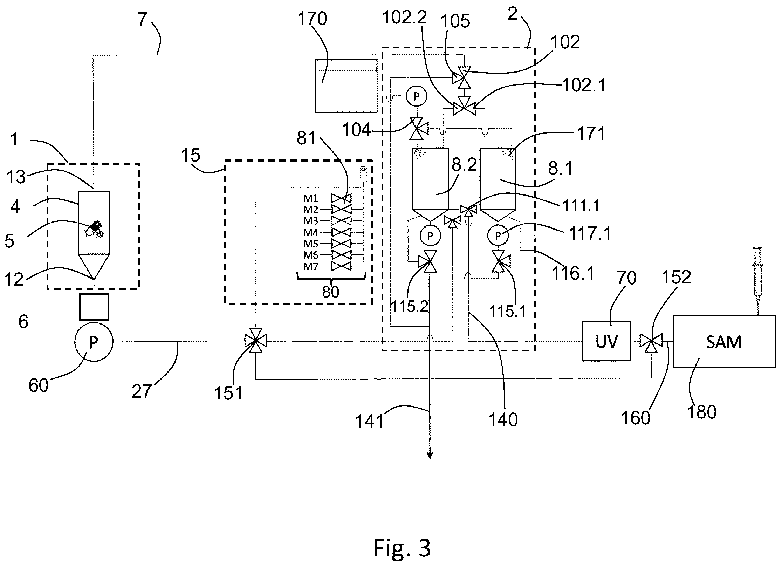

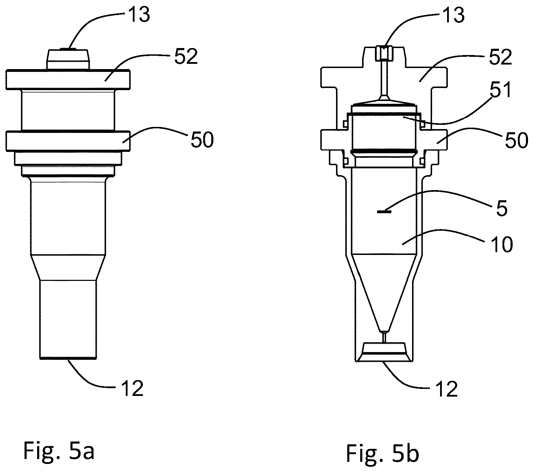

Figure 1 illustrates a setup of a dissolution testing apparatus as used in the present invention. The dissolution testing apparatus comprises a dissolution module 1, a sampling module 2 and an extraction module 3. The dissolution testing apparatus can be operated in a closed mode configuration, or an open mode configuration as will be explained below. The dissolution module 1 accommodates several dissolution cells designed as flow-through cells 4, that are lined up in a cell chamber 11 of the dissolution module 1. Each cell is filled with a dissoluble substance 5 (see Figure 5a and 5b). The flow-through cells 4 are supplied with dissolution medium by a dissolution medium reservoir 15 arranged in the dissolution module 1 or next to it, as shown in Figure 3. After passing the flow-through cells 4 the dissolution medium including dissolved substance exits the flow-through cells 4 as a substance solution containing dissolution medium and dissolved substance. The substance solution is transferred to the sampling module 2 via a substance solution conduit 7. In the sampling module 2 defined fractions of the substance solution are distributed from the substance solution conduit 7 into sampling vessels 8. Depending on the operation mode, the substance solution fractions in the sampling vessels can be tested and returned to their respective cell or the substance solution can be discharged to the extraction module 3 for testing or further processing, as will be explained below.

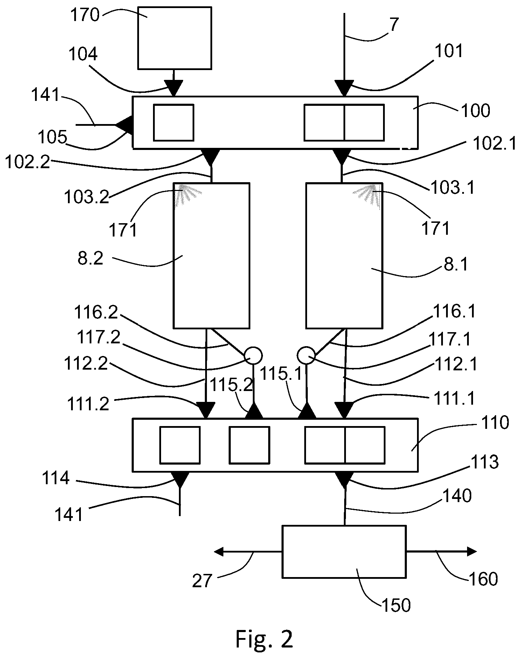

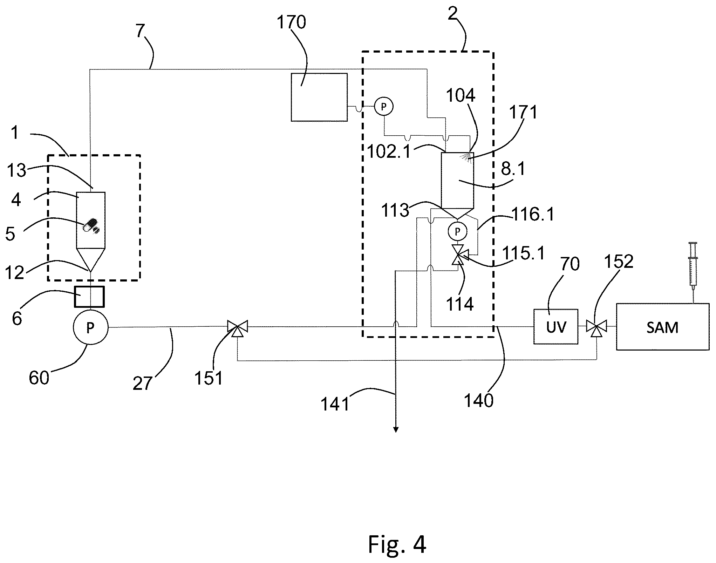

Figure 2 is a schematic diagram of the sampling module 2 of the dissolution testing apparatus, which comprises two sampling vessels 8.1 and 8.2 that are connectable to one of the several flow-through cells 4, an inlet fluid manifold 100 for selectively connecting the flow-through cell 4 to one of the two sampling vessels 8.1 or 8.2, and an outlet fluid manifold 110 for selectively discharging the selected sampling vessel 8.1, respectively 8.2, to a discharge line 140. The sampling module 2 may comprise a testing device for testing the substance solution of the selected sampling vessel. Alternatively, at least one testing device 70 can be arranged in the dissolution testing apparatus outside the sampling module 2 (see Figures 3 and 4). Further, the dissolution testing apparatus comprises a switch valve system 150 for selectively connecting the discharge line 140 to a cell inlet line 27 of the flow-through cell 4 or an extraction line 160.

The inlet fluid manifold 100 comprises an inlet port 101 in fluid communication with the substance solution conduit 7 of the flow-through cell 4 and an outlet port 102.1 and 102.2 for each of the sampling vessels 8.1 and 8.2, which are in fluid communication with a sampling vessel inlet line 103.1 and 103.2 leading to the respective sampling vessel 8.1 and 8.2. Further, the inlet fluid manifold 100 comprises a cleaning port 104, which is connected to a cleaning medium reservoir 170 located outside the at least two sampling vessels 8.1 and 8.2. The outlet port may be used to supply the cleaning medium to a spray nozzle 171 located inside each of the sampling vessels 8.1 and 8.2. The cleaning medium reservoir 170 and the spray nozzles 171 provide a cleaning system that is connected to the inlet fluid manifold to connect the cleaning system to the at least two sampling vessels 8.1 and 8.2. The cleaning system may be connected to both of the sampling vessels at the same time or may clean the sampling vessels selectively one after the other. Finally, the inlet fluid manifold 110 comprises a waste port 105 to discard substance solution before it reaches the sampling vessels.

The outlet fluid manifold 110 comprises an inlet port 111.1 and 111.2 for each of the sampling vessels 8.1 and 8.2, which are in fluid communication with a sampling vessel outlet line 112.1 and 112.2. The outlet fluid manifold 110 comprises an outlet port 113 in fluid communication with the discharge line 140. Further, the outlet fluid manifold 110 comprises a waste port 114 for connecting the sampling vessels 8.1 and 8.2 to a waste line 141 for discarding substance solution from the sampling vessels. Still further, the outlet fluid manifold 110 comprises a stirring port 115.1 and 115.2 for each sampling vessel for connecting the sampling vessels 8.1 and 8.2 to a stirring loop. The stirring loop includes the sampling vessel outlet line 112.1/112.2 and a loop line 116.1/116.2 comprising a pump 117.1/117.2 for pumping substance solution through the stirring loop back into the sampling vessel 8.1/8.2. The stirring loop provides a continuous stirring flow of the substance solution in the sampling vessels and allows for sorrow mixing of the substance solution content in the sampling vessel. With this setup, fractions of the substance solution collected in the sampling vessels advantageously are stirred in the stirring loop before being tested by the at least one testing device 70.

When the switch valve system connects the discharge line 140 to the extraction line 160, the dissolution testing apparatus is in an open loop configuration. In the open loop mode, for example the first sampling vessel 8.1 of the two sampling vessels is connected to the flow-through cell 4 via the inlet port 101 and the outlet port 102.1 of the inlet fluid manifold 100 and is filled with a first fraction of the substance solution. Then the sampling vessel 8.1 is disconnected from the flow-through cell 4 by closing the outlet port 102.1, so that the first fraction for example can be tested by a testing device. Meanwhile, the inlet fluid manifold 100 connects the second sampling vessel 8.2 of the two sampling vessels to the flow-through cell 4 using the outlet port 102.2 and the sampling vessel 8.2 is filled with a second fraction of the substance solution. The two sampling vessels 8.1 and 8.2 are alternately connected to the flow-through cell 4 and the discharge line 140 such that one sampling vessel is filled with substance solution while substance solution in the other sampling vessel is tested and/discharged, and vice versa. For example, the first sampling vessel 8.1 is discharged by the outlet fluid manifold 110 using the inlet port 111.1 and the outlet port 113 to drain the fraction of the sampling vessel 8.1 into the discharge line 140. Thus, the first fraction in the sampling vessel 8.1 can be discharged to the extraction line 160 after testing by the switch valve system 150, while the other sampling vessel 8.2 is filled with the second fraction of the substance solution for testing.

Advantageously, the solution flow rate and the timing of alternately switching connection between the two sampling vessels 8.1 and 8.2 and the flow-through cell 4 as well as the discharge line 140 is controlled such that a continuous substance solution flow is exiting from the flow-through cell 4. Also, the setup allows for modulating the substance solution flow if desired.

The dissolution testing apparatus comprises a controller that controls the operating conditions of the dissolution testing apparatus. The controller for example comprises a processing unit, a user interface and control display. The controller is particularly configured for controlling the inlet fluid manifold 100, the outlet fluid manifold 110 and the switch valve system 150 for steering the selective fluid connection of the flow-through cell 4 to the two sampling vessels 8.1 and 8.2. The controller for example receives measurement data from the pump device pumping dissolution medium and substance solution within the dissolution testing apparatus, from pressure sensors or gauges arranged in the dissolution medium and substance solution lines, from sample solution testing devices, like a UV spectrophotometer, or any other sensors used in the dissolution testing apparatus. For example, the controller receives a testing protocol for testing a specific dissoluble substance and controls the operation of the dissolution testing apparatus according to requirements of the testing protocol.

Figures 3 and 4 schematically illustrates a setup of the dissolution module 1 and the sampling module 2 of the dissolution testing apparatus. The flow-through cell 4 with a cell inlet 12 and a cell outlet 13 accommodates a dissoluble substance 5. The cell inlet 12 is connected to the cell inlet line 27, which is connected to the dissolution medium reservoir 15 by a first valve unit 151 for receiving dissolution medium. The dissolution medium reservoir 15 comprises a media selector 80 providing several different solvents and a media manifold 81, which is connectable to the flow-through cell 4 by the valve unit 151. The cell inlet line 27 comprises a pump device 60 and a dissolution medium heating module 6. The heating module 6 is arranged as part of the inlet line and advantageously heats the dissolution medium before the medium enters the flow-through cell 4. The cell outlet 13 is connected to the substance solution conduit 7, which transfers substance solution containing dissolution medium and dissolved substance from the flow-through cell 4 to the sampling module 2.

In the sampling module 2, the substance solution is alternately provided to the two sampling vessels 8.1 and 8.2 as described with respect to Figure 2. In the illustration according to Figures 3 and 4 the ports of the inlet fluid manifold 100 and the outlet fluid manifold 110 are depicted as switch valves to illustrate the optional connections of the fluid lines. The substance solution delivered by the substance solution conduit 7 is transferred to one of the sampling vessels 8.1 or 8.2, which is selected by the inlet fluid manifold 110. Alternatively, the substance solution can be directly transferred to the waste line 141 by the port 105 of the inlet fluid manifold 100. In the presented example, fractions of the substance solution from the sampling vessels 8.1 and 8.2 that are emitted to the discharge line 140 exit the sampling module 2 and are tested by the testing device 70. After that, the substance solution enters a second valve unit 152. The first valve unit 151 and the second valve unit 152 are both components of the switch valve system 150.

In the open loop mode, the second valve unit 152 connects the discharge line 140 to the extraction line 160, which transfers the substance solution to a sample manager 180 for collecting substance solution discharged from the sampling vessels 8.1 and 8.2 in sample solution vessels 181. As discussed earlier, the at least two sampling vessels of the dissolution testing apparatus, like the sampling vessels 8.1 and 8.2, provide flexible testing conditions and increase the through-put through the apparatus.

In Figure 4, the dissolution testing apparatus is operated in a closed loop mode. Components of the apparatus not necessarily involved in the closed loop dissolution operation are omitted to increase simplicity of the illustration. Particularly, the second sampling vessel is not shown because it is not required for the closed loop mode. However, the second sampling vessel does not need to be physically removed from the sampling module 2 as it can also be used for a pH change. In closed loop mode the sampling vessel is advantageously used as dissolution medium reservoir. The dissolution volume can be larger than the cell volume. The sampling vessel can be filled for example with 0.25, 0.5 or 1 litre and is used in this example as a bottle style reservoir. An extra dissolution medium reservoir, like mentioned before, is not required. In the second valve unit 152 of the switch valve system to substance solution is re-directed into a re-circle line 153 for transmitting the substance solution back to the first valve unit 151, which directs the substance solution back into the flow-through cell 4. In the closed loop mode, the same sampling vessel 8.1 can be used for the sampling of all of the substance solution samples required for testing. The discharge line 140 is connected to the cell inlet line 27 by the switch valve system 150 via the re-circling line 153. Several fractions can be sampled consecutively in the one sampling vessel and tested by the testing device.

The same dissolution testing apparatus can be used for open loop operation and closed mode operation. It is not necessary to modify the physical setup of the system, when switching from one mode to the other. The switch between the different operation modes can simply be controlled by the controller of the apparatus by steering the switch valve system. Thus, the dissolution testing apparatus according to the present invention saves time and resources and facilitates various differing testing procedures.

Figures 5a and 5b show a flow-through cell 4 as it can be used for the present invention in an exploded view and an assembled view. The flow-through cell 4 comprises a cell volume 10 having a generally cylindrical shape and a predefined size, depending on the cells, described in the pharmacopeia. The dissolution cell volume 10 is tapered towards the bottom end, which comprises the cell inlet 12, and is open at an upper end. A dissoluble substance 5 is placed in the cell. Holding structures, a bead bed or similar may be used inside the cell volume to place the dissoluble substance distanced from the bottom if required by a used testing method or a shape of a dissoluble substance. A closing ring 50 with a central through passage and a holding frame for a filter 51 is placed on the upper opening of the cell volume 10. The filter 51 can be selected according to testing requirements for the tested dissoluble substance. A cap 52 comprising the cell outlet 13 is mounted on the closing ring 50 to close the cell volume 10 and keep the filter 51 in place.

The dissolution testing apparatus according to the present invention can be further amended by implementing a heating module 6 for heating dissolution medium at the medium inlet line 27 to the flow-through cell 4 as described in the parallel patent application of the applicant having the title "Heating Module for Heating a Dissolution Medium of a Dissolution Testing Apparatus". Further, the dissolution testing apparatus according to the present invention can be further amended by implementing a heating channel for heating dissolution medium in the flow-through cell as described in the parallel patent application of the applicant having the title "Dissolution Testing Apparatus with a Common Heating Channel". The heating module and the heating channel allow for an uninterrupted fluid flow in the dissolution testing apparatus while the dissolution medium is heated to a required temperature. Therefore, the testing processes can be streamlined and adjusted to in vivo conditions.

**List of Reference Numbers**

| | | | |
|---|---|---|---|
| 1 | dissolution module | 100 | inlet fluid manifold |
| 2 | sampling module | 101 | inlet port |
| 3 | extraction module | 102.1, 102.2 | outlet port |
| 4 | flow-through cell | 103.1, 103.2 | inlet line |
| 5 | dissoluble substance | 104 | cleaning port |
| 6 | dissolution medium heating module | 110 | outlet fluid manifold |
| | | 111.1, 111.2 | inlet port |
| 7 | substance solution conduit | 112.1, 112.2 | outlet line |
| 8.1, 8.2 | sample vessel | 113 | outlet port |
| 10 | cell volume | 114 | waste port |
| 11 | cell chamber | 115.1, 115.2 | stirring port |
| 12 | cell inlet | 140 | discharge line |
| 13 | cell outlet | 141 | waste line |
| 15 | dissolution medium reservoir | 150 | switch valve system |
| 27 | cell inlet line | 151 | first valve unit |
| 29 | control unit | 152 | second valve unit |
| 50 | closing ring | 160 | extraction line |
| 51 | filter | 170 | cleaning medium |
| 52 | cap | 171 | spray nozzle |
| 60 | medium pump | 180 | sample manager |
| 70 | testing device | 181 | sample solution vessel |
| 80 | media selector | | |
| 81 | media manifold | | |

## Claims

1. Dissolution testing apparatus for testing a dissoluble substance dissolved in a dissolution medium comprising
- at least one dissolution medium reservoir (15),
- at least one flow-through cell (4) for receiving a dissoluble substance to be tested, which is connected to the dissolution medium reservoir (15),
- sampling vessels (8.1, 8.2) for sampling a substance solution containing dissolution medium and dissolved substance, and
- at least one pump device (60) for pumping dissolution medium through the at least one flow-through cell (4),
**characterized in that**, the dissolution testing apparatus further comprises
- at least two sampling vessels (8.1, 8.2) connectable to one flow-through cell (4),
- an inlet fluid manifold (100) for selectively connecting the flow-through cell (4) to one of the at least two sampling vessels (8.1, 8.2),
- an outlet fluid manifold (110) for selectively connecting one of the at least two sampling vessel (8.1, 8.2) to a discharge line (140),
and
- a switch valve system (150) for selectively connecting the discharge line (140) to a cell inlet line (27) of the flow-through cell (4) or an extraction line (160).

2. Dissolution testing apparatus according to claim 1, wherein the inlet fluid manifold (100), the outlet fluid manifold (110) and the switch valve system (150) are connected to a controller for controlling selective fluid connection.

3. Dissolution testing apparatus according to claim 1 or 2, wherein a testing device (70) is arranged at the discharge line and/or at the at least one sampling vessel (8.1, 8.2) for testing substance solution in the discharge line (140) and/or in the sampling vessels (8.1, 8.2).

4. Dissolution testing apparatus according to the preceding claims, wherein the inlet fluid manifold (100) comprises an inlet port (101) in fluid communication with a substance solution conduit (7) of the flow-through cell (4) and an outlet port (102.1, 102.2) for each of the sampling vessels (8.1, 8.2), which is in fluid communication with a sampling vessel inlet line (112.1, 112.2).

5. Dissolution testing apparatus according to the preceding claims, wherein the outlet fluid manifold (110) comprises an inlet port (111.1, 111.2) for each of the sampling vessels (8.1, 8.2), which are in fluid communication with a sampling vessel outlet line (112.1, 112.2) of the respective sampling vessel (8.1, 8.2), and an outlet port (113) in fluid communication with the discharge line (140).

6. Dissolution testing apparatus according to one of the preceding claims, wherein a stirring loop is provided at a sampling vessel outlet line (112.1, 112.2), comprising a pump (117.1, 117.2) for pumping substance solution through the stirring loop and back into the sampling vessel (8.1, 8.2).

7. Dissolution testing apparatus according to the preceding claims, wherein the outlet fluid manifold (110) comprises a stirring port (115.1, 115.2) for connecting the at least one sampling vessel (8.1, 8.2) to the stirring loop.

8. Dissolution testing apparatus according to one of the preceding claims, wherein the outlet fluid manifold (110) comprises a waste port (141) for connecting the at least one sampling vessel (8.1, 8.2) to a waste line (141).

9. Dissolution testing apparatus according to one of the preceding claims, wherein a cleaning system comprising a cleaning medium reservoir (170) outside the at least two sampling vessels (8.1, 8.2) and a spray nozzle (171) located in the sampling vessels (8.1, 8.2), wherein the cleaning system is connected to the inlet fluid manifold (100) to connect the cleaning system to the at least two sampling vessels (8.1, 8.2).

10. Dissolution testing apparatus according to one of the preceding claims, wherein the dissolution medium reservoir (15) is connectable to the flow-through cell (4) by the switch valve system. (150)

11. Dissolution testing apparatus according to one of the preceding claims, wherein the dissolution medium reservoir (15) comprises a media selector (80) providing several different solvents, and a media manifold (81), which is connectable to the flow-through cell (4) by the switch valve system (150).

12. Dissolution testing method for testing a dissoluble substance dissolved in a dissolution medium using a dissolution testing apparatus according to one of the previous claims, wherein the inlet fluid manifold (100), the outlet fluid manifold (110) and/or the switch valve system (150) establish selective connections such that
the apparatus is operated in an open loop mode by connecting the discharge line (140) to the extraction line (160) or the apparatus is operated in a closed loop mode by connecting the discharge line (140) to the cell inlet line (27).

13. Dissolution testing method according to claim 12, wherein the at least two sampling vessels (8.1, 8.2) are alternately connected to the flow-through cell (4) and the discharge line (140) such that a continuous substance solution flow is exiting in the flow-through cell (4).

14. Dissolution testing method according to one of the preceding claims 12 to 13, wherein in the open loop mode the at least two sampling vessels (8.1, 8.2) are alternately connected to the flow-through cell (4) and the discharge line (140) such that one sampling vessel (8.1) is filled with substance solution while substance solution in another sampling vessel (8.2) is tested and/or discharged.

15. Dissolution testing method according to one of the preceding claims 12 to 14, wherein in the open loop mode one sampling vessel (8.1) of the at least two sampling vessels (8.1, 8.2), that has been filled with a first fraction of the substance solution, is disconnected from the flow-through cell and the first fraction is tested by the testing device, while another sampling vessel (8.2) of the at least two sampling vessels (8.1, 8.2) is connected to the flow-through cell (4) and filled with a second fraction of the substance solution.

16. Dissolution testing method according to one of the preceding claims 12 to 15, wherein in a closed loop mode the flow-through cell (4) is connected to one of the at least two sampling vessels (8.1) and the discharge line (140) is connected to the cell inlet line (27), and several fractions are sampled consecutively in the one sampling vessel (8.1) and tested by the testing device (70).

17. Dissolution testing method according to one of the preceding claims 12 to 16, wherein the fractions are stirred in the stirring loop before being tested by the at least one testing device (70).

## Patentansprüche

1. Auflösungsprüfeinrichtung zum Prüfen einer auflösbaren Substanz, die in einem Auflösungsmedium gelöst ist, umfassend
- mindestens ein Auflösungsmedienreservoir (15),
- mindestens eine Durchflusszelle (4) zum Aufnehmen einer zu prüfenden auflösbaren Substanz, die mit dem Auflösungsmedienreservoir (15) verbunden ist,
- Probenahmegefäße (8.1, 8.2) zum Probennehmen einer Substanzlösung, die ein Auflösungsmedium und eine gelöste Substanz enthält, und
- mindestens eine Pumpenvorrichtung (60) zum Pumpen eines Auflösungsmediums durch die mindestens eine Durchflusszelle (4),
**dadurch gekennzeichnet, dass** die Auflösungsprüfeinrichtung ferner umfasst
- mindestens zwei Probenahmegefäße (8.1, 8.2), die mit einer Durchflusszelle (4) verbindbar sind,
- einen Einlassfluidverteiler (100) zum selektiven Verbinden der Durchflusszelle (4) mit einem der mindestens zwei Probenahmegefäße (8.1, 8.2),
- einen Auslassfluidverteiler (110) zum selektiven Verbinden eines der mindestens zwei Probenahmegefäße (8.1, 8.2) mit einer Abgabeleitung (140),
und
- ein Schaltventilsystem (150) zum selektiven Verbinden der Abgabeleitung (140) mit einer Zelleinlassleitung (27) der Durchflusszelle (4) oder einer Extraktionsleitung (160).

2. Auflösungsprüfeinrichtung nach Anspruch 1, wobei der Einlassfluidverteiler (100), der Auslassfluidverteiler (110) und das Schaltventilsystem (150) mit einer Steuerung zum Steuern der selektiven Fluidverbindung verbunden sind.

3. Auflösungsprüfeinrichtung nach Anspruch 1 oder 2, wobei eine Prüfvorrichtung (70) an der Abgabeleitung und/oder an dem mindestens einen Probenahmegefäß (8.1, 8.2) zum Prüfen der Substanzlösung in der Abgabeleitung (140) und/oder in den Probenahmegefäßen (8.1, 8.2) angeordnet ist.

4. Auflösungsprüfeinrichtung nach den vorstehenden Ansprüchen, wobei der Einlassfluidverteiler (100) eine Einlassöffnung (101) in Fluidverbindung mit einer Substanzlösungsleitung (7) der Durchflusszelle (4) und eine Auslassöffnung (102.1, 102.2) für jedes der Probenahmegefäße (8.1, 8.2) umfasst, die in Fluidverbindung mit einer Probenahmegefäßeinlassleitung (112.1, 112.2) steht.

5. Auflösungsprüfeinrichtung nach den vorstehenden Ansprüchen, wobei der Auslassfluidverteiler (110) eine Einlassöffnung (111.1, 111.2) für jedes der Probenahmegefäße (8.1, 8.2), die in Fluidverbindung mit einer Probenahmegefäßauslassleitung (112.1, 112.2) des jeweiligen Probenahmegefäßes (8.1, 8.2) stehen, und eine Auslassöffnung (113) in Fluidverbindung mit der Abgabeleitung (140) umfasst.

6. Auflösungsprüfeinrichtung nach einem der vorstehenden Ansprüche, wobei eine Rührschleife an einer Probenahmegefäßauslassleitung (112.1, 112.2) bereitgestellt ist, umfassend eine Pumpe (117.1, 117.2) zum Pumpen der Substanzlösung durch die Rührschleife und zurück in das Probenahmegefäß (8.1, 8.2).

7. Auflösungsprüfeinrichtung nach den vorstehenden Ansprüchen, wobei der Auslassfluidverteiler (110) eine Rühröffnung (115.1, 115.2) zum Verbinden des mindestens einen Probenahmegefäßes (8.1, 8.2) mit der Rührschleife umfasst.

8. Auflösungsprüfeinrichtung nach einem der vorstehenden Ansprüche, wobei der Auslassfluidverteiler (110) eine Abfallöffnung (141) zum Verbinden des mindestens einen Probenahmegefäßes (8.1, 8.2) mit einer Abfallleitung (141) umfasst.

9. Auflösungsprüfeinrichtung nach einem der vorstehenden Ansprüche, wobei ein Reinigungssystem, umfassend ein Reinigungsmedienreservoir (170) außerhalb der mindestens zwei Probenahmegefäße (8.1, 8.2) und eine Sprühdüse (171), die in den Probenahmegefäße (8.1, 8.2) gelegen ist, wobei das Reinigungssystem mit dem Einlassfluidverteiler (100) verbunden ist, um das Reinigungssystem mit den mindestens zwei Probenahmegefäßen (8.1, 8.2) zu verbinden.

10. Auflösungsprüfeinrichtung nach einem der vorstehenden Ansprüche, wobei das Auflösungsmedienreservoir (15) durch das Schaltventilsystem mit der Durchflusszelle (4) verbindbar ist. (150)

11. Auflösungsprüfeinrichtung nach einem der vorstehenden Ansprüche, wobei das Auflösungsmedienreservoir (15) einen Medienselektor (80), der mehrere verschiedene Lösungsmittel bereitstellt, und einen Medienverteiler (81) umfasst, der durch das Schaltventilsystem (150) mit der Durchflusszelle (4) verbindbar ist.

12. Auflösungsprüfeinrichtung zum Prüfen einer auflösbaren Substanz, die in einem Auflösungsmedium gelöst ist, unter Verwendung einer Auflösungsprüfeinrichtung nach einem der vorstehenden Ansprüche, wobei der Einlassfluidverteiler (100), der Auslassfluidverteiler (110) und/oder das Schaltventilsystem (150) selektive Verbindungen derart herstellen, dass
die Einrichtung in einem offenen Schleifenmodus betrieben wird, durch Verbinden der Abgabeleitung (140) mit der Extraktionsleitung (160), oder die Einrichtung in einem geschlossenen Schleifenmodus betrieben wird, durch Verbinden der Abgabeleitung (140) mit der Zelleinlassleitung (27).

13. Auflösungsprüfverfahren nach Anspruch 12, wobei die mindestens zwei Probenahmegefäße (8.1, 8.2) abwechselnd mit der Durchflusszelle (4) und der Abgabeleitung (140) derart verbunden sind, dass ein kontinuierlicher Substanzlösungsfluss in der Durchflusszelle (4) austritt.

14. Auflösungsprüfverfahren nach einem der vorstehenden Ansprüche 12 bis 13, wobei in dem offenen Schleifenmodus die mindestens zwei Probenahmegefäße (8.1, 8.2) abwechselnd mit der Durchflusszelle (4) und der Abgabeleitung (140) derart verbunden sind, dass ein Probenahmegefäß (8.1) mit Substanzlösung gefüllt wird, während die Substanzlösung in einem anderen Probenahmegefäß (8.2) geprüft und/oder abgegeben wird.

15. Auflösungsprüfverfahren nach einem der vorstehenden Ansprüche 12 bis 14, wobei in dem offenen Schleifenmodus ein Probenahmegefäß (8.1) der mindestens zwei Probenahmegefäße (8.1, 8.2), das mit einer ersten Fraktion der Substanzlösung gefüllt worden ist, von der Durchflusszelle getrennt wird und die erste Fraktion durch die Prüfvorrichtung geprüft wird, während ein anderes Probenahmegefäß (8.2) der mindestens zwei Probenahmegefäße (8.1, 8.2) mit der Durchflusszelle (4) verbunden ist und mit einer zweiten Fraktion der Substanzlösung gefüllt wird.

16. Auflösungsprüfverfahren nach einem der vorstehenden Ansprüche 12 bis 15, wobei in einem geschlossenen Schleifenmodus die Durchflusszelle (4) mit einem der mindestens zwei Probenahmegefäße (8.1) verbunden ist und die Abgabeleitung (140) mit der Zelleinlassleitung (27) verbunden ist, und mehrere Fraktionen nacheinander in dem einen Probenahmegefäß (8.1) entnommen und durch die Prüfvorrichtung (70) geprüft werden.

17. Auflösungsprüfverfahren nach einem der vorstehenden Ansprüche 12 bis 16, wobei die Fraktionen in der Rührschleife gerührt werden, bevor sie durch die mindestens eine Prüfvorrichtung (70) geprüft werden.

## Revendications

1. Appareil d'essai de dissolution destiné à tester une substance dissoluble dissoute dans un milieu de dissolution comprenant
- au moins un réservoir de milieu de dissolution (15),
- au moins une cellule de passage (4) destinée à recevoir une substance dissoluble à tester, qui est raccordée au réservoir de milieu de dissolution (15),
- des récipients d'échantillonnage (8.1, 8.2) pour l'échantillonnage d'une solution de substance contenant un milieu de dissolution et une substance dissoute, et
- au moins un dispositif de pompage (60) pour pomper le milieu de dissolution à travers au moins une cellule de circulation (4),
**caractérisé en ce que** l'appareil d'essai de dissolution comprend en outre
- au moins deux récipients d'échantillonnage (8.1, 8.2) pouvant être raccordés à une cellule de circulation (4),
- un collecteur de fluide d'entrée (100) pour raccorder sélectivement la cellule à circulation continue (4) à l'au moins un des deux récipients d'échantillonnage (8.1, 8.2),
- un collecteur de fluide de sortie (110) pour raccorder sélectivement l'au moins un des deux récipients d'échantillonnage (8.1, 8.2) à une conduite d'évacuation (140),
et
- un système de vanne de commutation (150) pour raccorder sélectivement la conduite d'évacuation (140) à une conduite d'entrée de cellule (27) de la cellule à circulation (4) ou à une conduite d'extraction (160).

2. Appareil d'essai de dissolution selon la revendication 1, dans lequel le collecteur de fluide d'entrée (100), le collecteur de fluide de sortie (110) et le système de vanne de commutation (150) sont raccordés à un dispositif de commande pour commander le raccordement sélectif des fluides.

3. Appareil d'essai de dissolution selon la revendication 1 ou 2, dans lequel un dispositif d'essai (70) est disposé sur la conduite d'évacuation et/ou sur au moins un récipient d'échantillonnage (8.1, 8.2) pour tester la solution de substance dans la conduite d'évacuation (140) et/ou dans les récipients d'échantillonnage (8.1, 8.2).

4. Appareil d'essai de dissolution selon les revendications précédentes, dans lequel le collecteur de fluide d'entrée (100) comprend un orifice d'entrée (101) en communication fluide avec un conduit de solution de substance (7) de la cellule à circulation (4) et un orifice de sortie (102.1, 102.2) pour chacun des récipients d'échantillonnage (8.1, 8.2), qui est en communication fluide avec une conduite d'entrée de récipient d'échantillonnage (112.1, 112.2).

5. Appareil d'essai de dissolution selon les revendications précédentes, dans lequel le collecteur de fluide de sortie (110) comprend un port d'entrée (111.1, 111.2) pour chacun des récipients d'échantillonnage (8.1, 8.2), qui sont en communication fluide avec une conduite de sortie de récipient d'échantillonnage (112.1, 112.2) du récipient d'échantillonnage respectif (8.1, 8.2), et un port de sortie (113) en communication fluide avec la conduite d'évacuation (140).

6. Appareil d'essai de dissolution selon l'une quelconque des revendications précédentes, dans lequel une boucle d'agitation est prévue à la conduite de sortie du récipient d'échantillonnage (112.1, 112.2), comprenant une pompe (117.1, 117.2) pour pomper la solution de substance à travers la boucle d'agitation et la renvoyer dans le récipient d'échantillonnage (8.1, 8.2).

7. Appareil d'essai de dissolution selon les revendications précédentes, dans lequel le collecteur de fluide de sortie (110) comprend un port d'agitation (115.1, 115.2) pour raccorder l'au moins un récipient d'échantillonnage (8.1, 8.2) à la boucle d'agitation.

8. Appareil d'essai de dissolution selon l'une quelconque des revendications précédentes, dans lequel le collecteur de fluide de sortie (110) comprend un orifice d'évacuation (141) pour raccorder l'au moins un récipient d'échantillonnage (8.1, 8.2) à une conduite d'évacuation (141).

9. Appareil d'essai de dissolution selon l'une quelconque des revendications précédentes, dans lequel un système de nettoyage comprend un réservoir de produit de nettoyage (170) à l'extérieur des au moins deux récipients d'échantillonnage (8.1, 8.2) et une buse de pulvérisation (171) située dans les récipients d'échantillonnage (8.1, 8.2), dans lequel le système de nettoyage est relié au collecteur de fluide d'entrée (100) pour raccorder le système de nettoyage aux au moins deux récipients d'échantillonnage (8.1, 8.2).

10. Appareil d'essai de dissolution selon l'une quelconque des revendications précédentes, dans lequel le réservoir de milieu de dissolution (15) peut être raccordé à la cellule à circulation (4) par le système de vanne de commutation. (150)

11. Appareil d'essai de dissolution selon l'une quelconque des revendications précédentes, dans lequel le réservoir de milieu de dissolution (15) comprend un sélecteur de milieu (80) fournissant plusieurs solvants différents, et un collecteur de milieu (81), qui est raccordé à la cellule à circulation (4) par le système de vanne de commutation (150).

12. Procédé d'essai de dissolution destiné à tester une substance dissoute dans un milieu de dissolution à l'aide d'un appareil d'essai de dissolution selon l'une quelconque des revendications précédentes, dans lequel le collecteur de fluide d'entrée (100), le collecteur de fluide de sortie (110) et/ou le système de vanne de commutation (150) établissent des raccordements sélectifs de telle sorte que
l'appareil fonctionne en boucle ouverte en reliant la conduite d'évacuation (140) à la conduite d'extraction (160) ou l'appareil fonctionne en boucle fermée en raccordant la conduite d'évacuation (140) à la conduite d'entrée de la cellule (27).

13. Procédé d'essai de dissolution selon la revendication 12, dans lequel les au moins deux récipients d'échantillonnage (8.1, 8.2) sont alternativement reliés à la cellule à circulation (4) et à la conduite d'évacuation (140) de manière à ce qu'un flux continu de solution de substance s'écoule dans la cellule à circulation (4).

14. Procédé d'essai de dissolution selon l'une quelconque des revendications précédentes 12 à 13, dans lequel, en mode boucle ouverte, les au moins deux récipients d'échantillonnage (8.1, 8.2) sont alternativement reliés à la cellule de passage (4) et à la conduite d'évacuation (140) de sorte qu'un récipient d'échantillonnage (8.1) est rempli de solution de substance tandis que la solution de substance dans un autre récipient d'échantillonnage (8.2) est testée et/ou déchargée.

15. Procédé d'essai de dissolution selon l'une quelconque des revendications précédentes 12 à 14, dans lequel, en mode boucle ouverte, un récipient d'échantillonnage (8.1) parmi les deux récipients d'échantillonnage au moins (8.1, 8.2), qui a été rempli d'une première fraction de la solution de substance, est déconnecté de la cellule à circulation et la première fraction est testée par le dispositif d'essai, tandis qu'un autre récipient d'échantillonnage (8.2) parmi les deux récipients d'échantillonnage au moins (8.1, 8.2) est raccordé à la cellule à circulation (4) et rempli d'une seconde fraction de la solution de substance.

16. Procédé d'essai de dissolution selon l'une quelconque des revendications précédentes 12 à 15, dans lequel, dans un mode en boucle fermée, la cellule à circulation (4) est raccordée à l'un des au moins deux récipients d'échantillonnage (8.1) et la conduite d'évacuation (140) est raccordée à la conduite d'entrée de la cellule (27), et plusieurs fractions sont échantillonnées consécutivement dans le seul récipient d'échantillonnage (8.1) et testées par le dispositif d'essai (70).

17. Procédé d'essai de dissolution selon l'une quelconque des revendications précédentes 12 à 16, dans lequel les fractions sont agitées dans la boucle d'agitation avant d'être testées par le au moins un dispositif d'essai (70).
